(19) Europäisches Patentamt
European Patent Office
Office européen des brevets

(11) Veröffentlichungsnummer: **0 453 842 A2**

(12) # EUROPÄISCHE PATENTANMELDUNG

(21) Anmeldenummer: **91105452.6**

(22) Anmeldetag: **06.04.91**

(51) Int. Cl.5: **C07C 69/712**, C07C 67/31, A01N 39/02, C07C 59/125, C07C 51/09, C07C 51/60, C07C 43/295, C07C 41/16, C07C 239/20, C07C 327/28, C07D 307/42

(30) Priorität: **21.04.90 DE 4012711**

(43) Veröffentlichungstag der Anmeldung: **30.10.91 Patentblatt 91/44**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Andree, Roland, Dr.**
**Schillerstrasse 17**
**W-4018 Langenfeld(DE)**
Erfinder: **Haug, Michael, Dr.**
**Fahner Weg 5**
**W-5060 Bergisch Gladbach 2(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Fluor-substituierte alpha-(5-Aryloxy-naphthalin-1-yl-oxy)-propionsäure-Derivate.**

(57) Beschrieben werden neue fluor-substituierte α-(5-Aryloxy-naphthalin-1-yl-oxy)-propionsäure-Derivate der Formel (I)

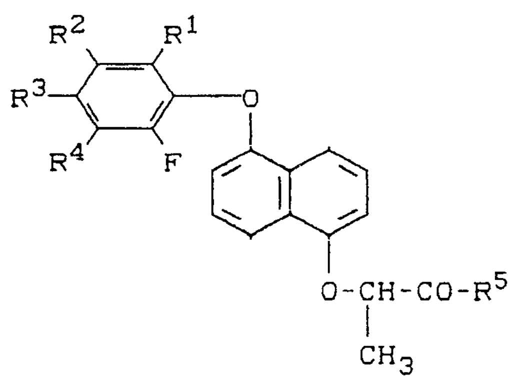

(I)

in welcher

$R^1$-$R^5$ die in der Beschreibung angegebene Bedeutung haben sowie Verfahren und neue Zwischenprodukte zu ihrer Herstellung.

Verwendet werden die neuen Verbindungen der Formel (II) als Herbizide.

EP 0 453 842 A2

Die Erfindung betrifft neue fluor-substituierte α-(5-Aryloxy-naphthalin-1-yl-oxy)-propionsäure-Derivate, Verfahren und neue Zwischenprodukte zu ihrer Herstellung sowie ihre Verwendung als Herbizide.

Es ist bereits bekannt, daß bestimmte α-(5-Aryloxy-naphthalin-1-yl-oxy)-propionsäure-Derivate, wie z.B. α-(5-(4-Trifluormethyl-phenoxy)-naphthalin-1-yl-oxy)-propionsäure-ethylester, herbizid wirksam sind (vgl. EP-A 309 864/LeA 25562). Die Wirkung dieser bekannten Verbindungen gegen Unkräuter sowie ihre Kulturpflanzen-Verträglichkeit sind jedoch nicht immer ganz zufriedenstellend.

Es wurden nun neue fluor-substituierte α-(5-Aryloxynaphthalin-1-yl-oxy)-propionsäure-Derivate der allgemeinen Formel (I)

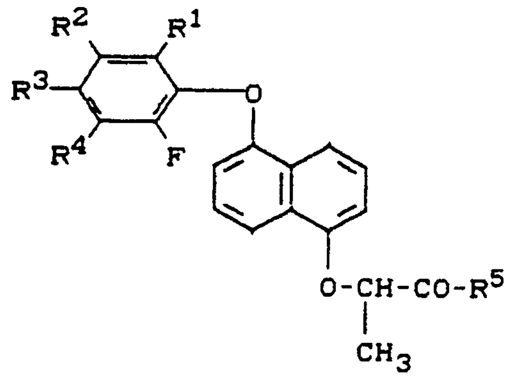

(I)

in welcher

$R^1$ für Wasserstoff, Fluor oder Cyano steht,

$R^2$ für Wasserstoff, Fluor oder Chlor steht,

$R^3$ für Fluor, Chlor, Cyano, Trifluormethyl oder Trifluormethylsulfonyl steht,

$R^4$ für Wasserstoff, Fluor oder Chlor steht und

$R^5$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, N-($C_1$-$C_6$-Alkoxy)-N-($C_1$-$C_4$-alkyl)-amino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxycarbonyl-$C_1$-$C_2$-alkylthio oder für die Gruppierung -O-$R^6$ steht, worin

$R^6$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxycarbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino-oxy-$C_1$-$C_4$-alkyl, $C_2$-$C_4$-Alkylidenamino oder für ein Ammonium-, ein $C_1$-$C_4$-Alkylammonium-, ein Natrium-, Kalium-oder Calcium-äquivalent steht, oder für die Gruppierung

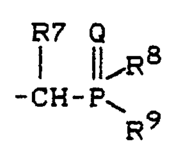

steht, worin

$R^7$ für Wasserstoff, $C_1$-$C_4$-Alkyl, Phenyl, Furyl, Thienyl oder Pyridyl steht,

$R^8$ für $C_1$-$C_4$-Alkyl oder $C_1$-$C_4$-Alkoxy steht,

$R^9$ für $C_1$-$C_4$-Alkoxy steht und

Q für Sauerstoff oder Schwefel steht,

oder

$R^6$ für die Gruppierung $-(CH_2)_n-R^{10}$ steht, worin

n für die Zahlen 0, 1 oder 2 steht und

$R^{10}$ für einen gegebenenfalls durch Fluor, Chlor, Brom und/oder $C_1$-$C_4$-Alkyl substituierten heterocy-

clischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Isoxazolidinyl, Pyridinyl oder Pyrimidinyl steht,

gefunden.

Die Verbindungen der Formel (I) enthalten mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Weiter wurde gefunden, daß man die neuen fluor-substituierten α-(5-Aryloxy-naphthalin-1-yl-oxy)-propionsäure-Derivate der Formel (I) erhält, wenn man

(a) fluor-substituierte 5-Aryloxy-1-naphthole der allgemeinen Formel (II)

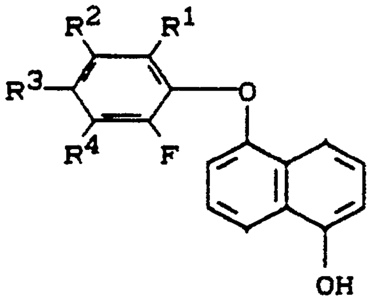

(II)

in welcher

$R^1$, $R^2$, $R^3$ und $R^4$ die oben angegebenen Bedeutungen haben,

mit Propionsäure-Derivaten der allgemeinen Formel (III)

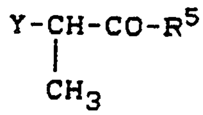

(III)

in welcher

$R^5$ die oben angegebene Bedeutung hat und

Y für eine nucleophile Abgangsgruppe steht,

gegebenenfalls in Gegenwart eines Säureakzeptors und gegebenenfalls in Gegenwart eines Verdünnungsmittels umsetzt, oder

(b) Halogen-benzol-Derivate der allgemeinen Formel (IV)

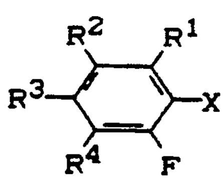

(IV)

in welcher

$R^1$, $R^2$, $R^3$ und $R^4$ die oben angegebenen Bedeutungen haben und

X für Halogen steht,

mit α-(5-Hydroxy-naphthalin-1-yl-oxy)-propionsäure-Derivaten der allgemeinen Formel (V)

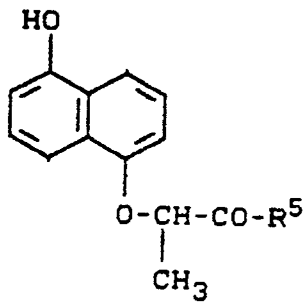

(V)

in welcher

$R^5$ die oben angegebene Bedeutung hat,

gegebenenfalls in Gegenwart eines Säureakzeptors und gegebenenfalls in Gegenwart eines Verdünnungsmittels umsetzt, oder

(c) für den Fall, daß $R^5$ für Hydroxy steht und die Reste $R^1$ bis $R^4$ die oben angegebenen Bedeutungen haben, Verbindungen der allgemeinen Formel (I), in welcher $R^5$ für Methoxy oder Ethoxy steht und die Reste $R^1$ bis $R^4$ die oben angegebenen Bedeutungen haben,

mit einem Alkalihydroxid in Gegenwart von Wasser und gegebenenfalls in Gegenwart eines organischen Lösungsmittels umsetzt und dann - gegebenenfalls nach Einengen - mit einer Mineralsäure ansäuert, oder

(d) für den Fall, daß $R^5$ für Chlor steht und die Reste $R^1$ bis $R^4$ die oben angegebenen Bedeutungen haben, Verbindungen der allgemeinen Formel (I), in welcher $R^5$ für Hydroxy steht und die Reste $R^1$ bis $R^4$ die oben angegebenen Bedeutungen haben,

mit einem Chlorierungsmittel gegebenenfalls in Gegenwart eines Katalysators und gegebenenfalls in Gegenwart eines Verdünnungsmittels umsetzt, oder

(e) für den Fall, daß $R^5$ mit Ausnahme von Chlor die oben angegebene Bedeutung hat und die Reste $R^1$ bis $R^4$ die oben angegebenen Bedeutungen haben, Verbindungen der allgemeinen Formel (I), in welcher $R^5$ für Chlor steht und die Reste $R^1$ bis $R^4$ die oben angegebenen Bedeutungen haben,

mit Verbindungen der allgemeinen Formel (VI)

$H\text{-}R^5$ (VI)

in welcher

$R^5$ mit Ausnahme von Chlor die oben angegebene Bedeutung hat,

gegebenenfalls in Gegenwart eines Säureakzeptors und gegebenenfalls in Gegenwart eines Verdünnungsmittels umsetzt, oder

(f) für den Fall, daß $R^5$ für die Gruppierung $\text{-O-}R^6$ steht, worin $R^6$ mit Ausnahme von Ammonium, Alkylammonium, Alkalimetall und Erdalkalimetall die oben angegebene Bedeutung hat und die Reste $R^1$ bis $R^4$ die oben angegebenen Bedeutungen haben, Verbindungen der allgemeinen Formel (I), in welcher $R^5$ für Hydroxy steht und die Reste $R^1$ bis $R^4$ die oben angegebenen Bedeutungen haben,

mit Hydroxyverbindungen der allgemeinen Formel (VII)

$HO\text{-}R^6$ (VII)

in welcher

$R^6$ mit Ausnahme von Ammonium, Alkylammonium, Alkalimetall und Erdalkalimetall die oben angegebenen Bedeutungen hat,

gegebenenfalls in Gegenwart eines Katalysators und gegebenenfalls in Gegenwart eines Verdünnungsmittels umsetzt.

Schließlich wurde gefunden, daß die neuen fluor-substituierten α-(5-Aryloxy-naphthalin-1-yl-oxy)-propionsäure-Derivate der allgemeinen Formel (I) ausgezeichnete herbizide Eigenschaften aufweisen.

Überraschenderweise zeigen die erfindungsgemäßen fluorsubstituierten α-(5-Aryloxy-naphthalin-1-yl-oxy)-propionsäure-Derivate der Formel (I) bei guter Kulturpflanzen-Verträglichkeit gegen bestimmte Problemunkräuter wesentlich stärkere Wirkung als α-(5-(4-Trifluormethylphenoxy)-naphthalin-1-yl-oxy)-propionsäure-ethylester, welches ein strukturell ähnlicher vorbekannter Wirkstoff gleicher Wirkungsrichtung ist.

Die Erfindung betrifft vorzugsweise Verbindungen der Formel (I), in welcher

$R^1$ für Wasserstoff oder Fluor steht,

$R^2$ für Wasserstoff oder Fluor steht,

$R^3$ für Trifluormethyl oder Trifluormethylsulfonyl steht,

$R^4$ für Wasserstoff oder Fluor steht und

$R^5$ für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, N-($C_1$-$C_4$-Alkoxy)-N-($C_1$-$C_3$-alkyl)-amino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung -O-$R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl $C_2$-$C_4$-Alkylidenamino-oxy-$C_2$-$C_3$-alkyl oder für ein Ammonium-, $C_1$-$C_3$-Alkylammonium-, Natrium-, oder Kalium-äquivalent steht,

oder für die Gruppierung

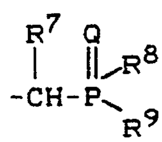

steht,

worin

$R^7$ für Wasserstoff, Methyl, Phenyl, Furyl, Thienyl oder Pyridyl steht,

$R^8$ für Methoxy oder Ethoxy steht,

$R^9$ für Methoxy oder Ethoxy steht und

Q für Sauerstoff oder Schwefel steht

oder

R [6] für die Gruppierung (-$CH_2$-$)_n$-$R^{10}$ steht, worin

n für die Zahlen 0, 1 oder 2 steht und

$R^{10}$ für einen gegebenenfalls durch Chlor und/oder Methyl subtituierten heterocyclischen Rest aus der Reihe Furyl, Tetrahydrofuryl, Thienyl, Perhydropyranyl, Oxazolyl, Thiazolyl, Isoxazolidinyl und Dioxolanyl steht.

Die Erfindung betrifft insbesondere Verbindungen der Formel (I), in welcher

$R^1$, $R^2$ und $R^4$ für Wasserstoff stehen,

$R^3$ für Trifluormethyl steht und

$R^5$ für Chlor, Hydroxy, Methoxy, Ethoxy, Propoxy, Isopropoxy, Butoxy, Isobutoxy, sec-Butoxy, t-Butoxy, Methoxyethoxy, Ethoxyethoxy, Benzyloxyethoxy, Benzyloxypropoxy, Methoxycarbonylmethoxy, Ethoxycarbonylmethoxy, Methoxycarbonylethoxy, iso-Propylidenamino-oxy-ethoxy oder Ethoxycarbonylethoxy steht.

Die R-Isomeren der insbesondere bevorzugten Verbindungen der Formel (I) werden ganz besonders bevorzugt.

Verwendet man für das erfindungsgemäße Verfahren (a) beispielsweise 5-(2-Fluor-4-trifluormethyl-phenoxy)-1-naphthol und $\alpha$-Brom-propionsäure-ethylester als Ausgangsstoffe, so kann der Reaktionsablauf durch das folgende Formelschema wiedergegeben werden:

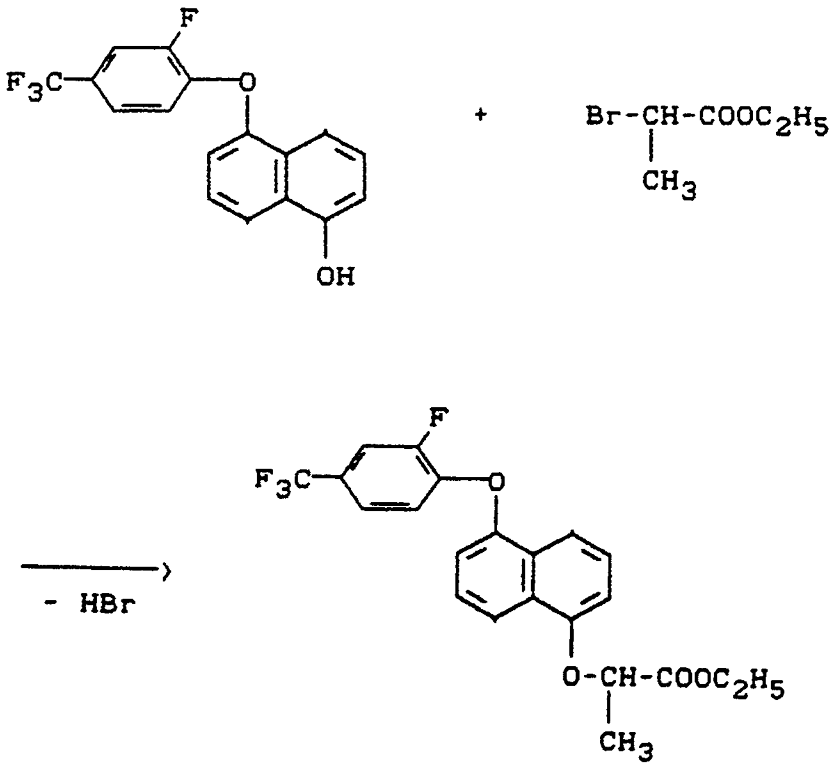


Verwendet man für das erfindungsgemäße Verfahren (b) beispielsweise 3,4,5-Trifluor-benzotrifluorid und α-(5-Hydroxy-naphthalin-1-yl-oxy)-propionsäure-propylester als Ausgangsstoffe, so kann der Reaktionsablauf durch das folgende Formelschema wiedergegeben werden:

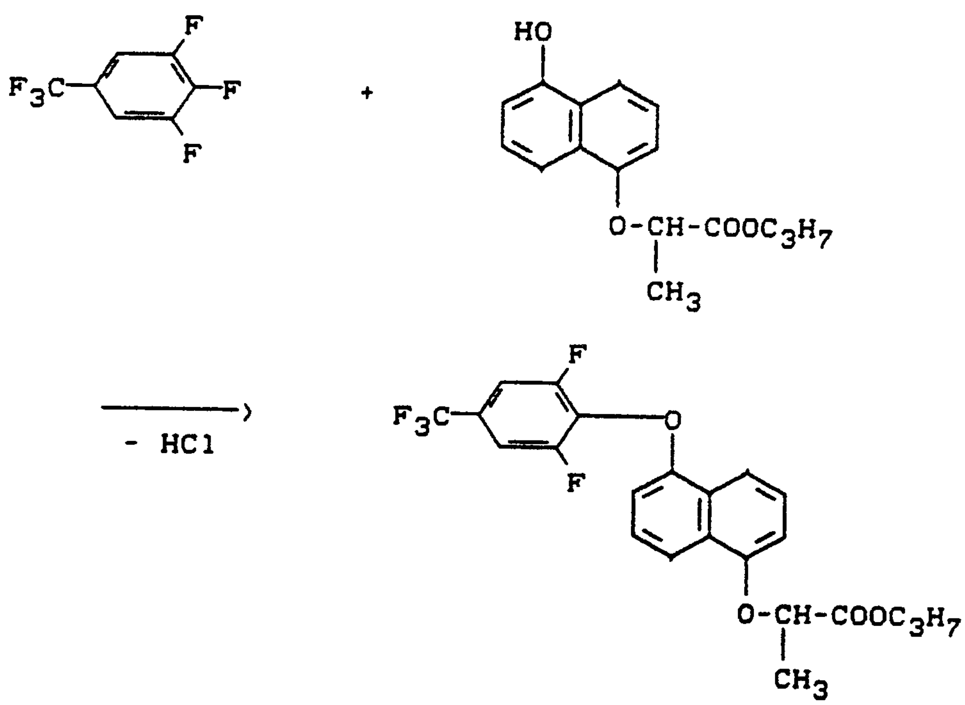


Verwendet man für das erfindungsgemäße Verfahren (c) beispielsweise α-(5-(2-Fluor-4-trifluormethyl-phenoxy)-naphthalin-1-yl-oxy)-propionsäure-methylester und Natronlauge als Ausgangsstoffe, so kann der

Reaktionsablauf durch das folgende Formelschema wiedergegeben werden:

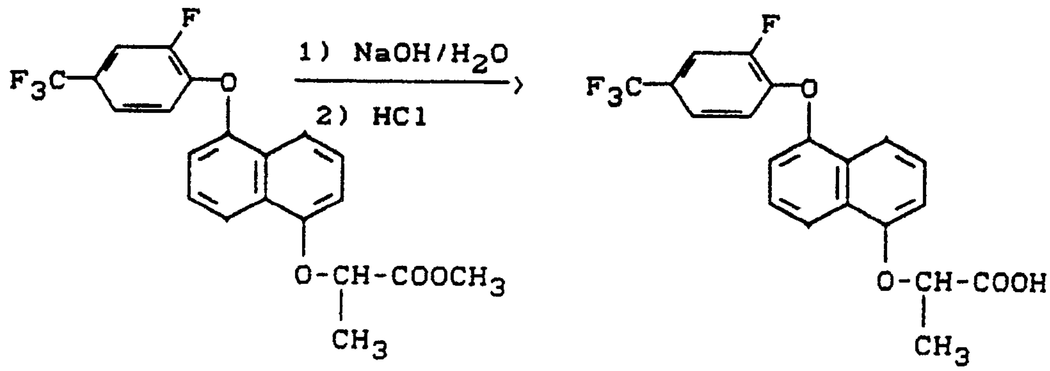


Verwendet man für das erfindungsgemäße Verfahren (d) beispielsweise α-(5-(2,3,6-Trifluor-4-trifluormethylphenoxy)-naphthalin-1-yl-oxy)-propionsäure und Thionylchlorid als Ausgangsstoffe, so kann der Reaktionsablauf durch das folgende Formelschema wiedergegeben werden:

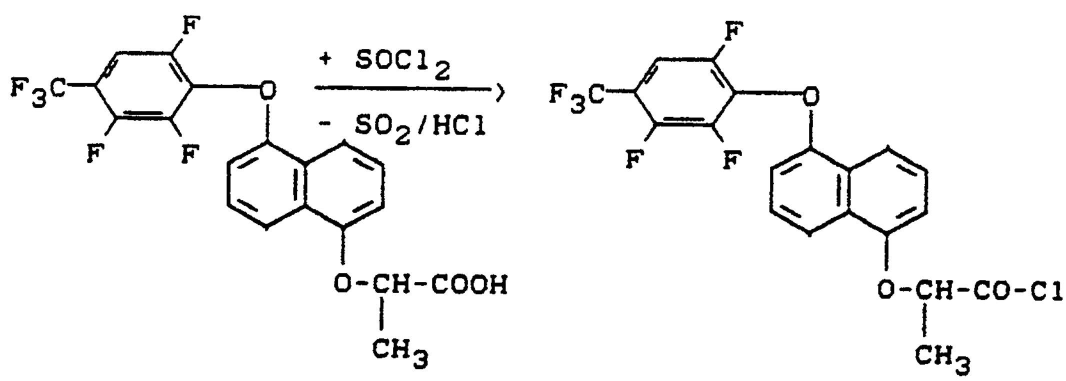


Verwendet man für das erfindungsgemäße Verfahren (e) beispielsweise α-(5-(2,6-Difluor-4-trifluormethylphenoxy)-naphthalin-1-yl-oxy)-propionsäure-chlorid und Butanol als Ausgangsstoffe, so kann der Reaktionsablauf duch das folgende Formelschema wiedergegeben werden:

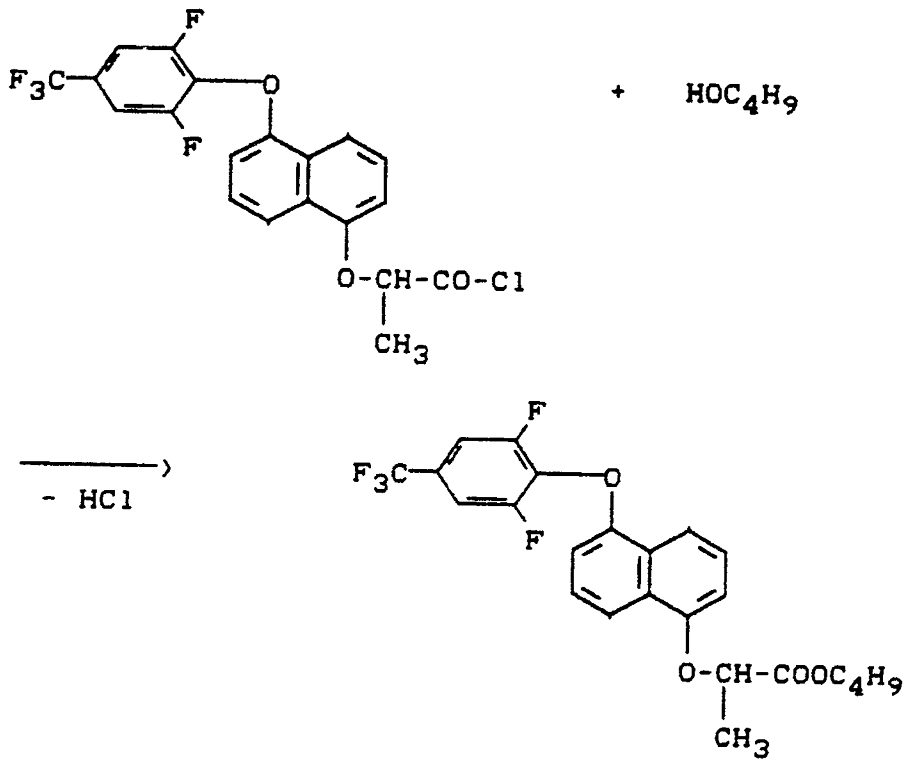


Verwendet man für das erfindungsgemäße Verfahren (f) beispielsweise α-(5-(2,3,6-Trifluor-4-trifluormethylphenoxy)-naphthalin-1-yl-oxy)-propionsäure und Isopropanol als Ausgangsstoffe, so kann der Reaktionsablauf durch das folgende Formelschema wiedergegeben werden:

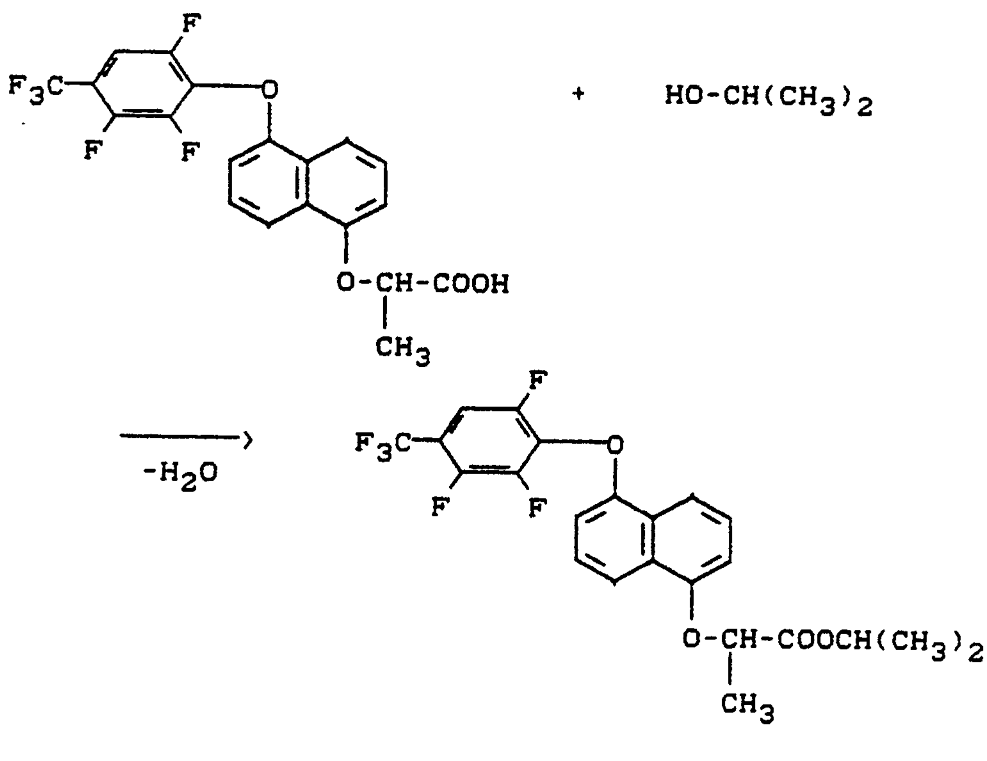


Die beim erfindungsgemäßen Verfahren (a) zur Herstellung von Verbindungen der Formel (I) als Ausgangsstoffe zu verwendenden 5-Aryloxy-1-naphthole sind durch die Formel (II) allgemein definiert.

In Formel (II) haben $R^1$, $R^2$, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$ und $R^4$ angegeben wurden.

Als Beispiele für die Ausgangsstoffe der Formel (II) seien genannt:
5-(2-Fluor-4-trifluormethyl-phenoxy)-1-naphthol, 5-(2,6-Difluor-4-trifluormethyl-phenoxy)-1-naphthol, 5-(2-Cyano-6-fluor-4-trifluormethyl-phenoxy-1-naphthol, und 5-(2,3,6-Trifluor-4-trifluormethyl-phenoxy)-1-naphthol.

Die Ausgangsstoffe der Formel (II) sind noch nicht aus der Literatur bekannt. Man erhält die Verbindungen der Formel (II), wenn man entsprechende Halogen-benzol-Derivate der allgemeinen Formel (IV)

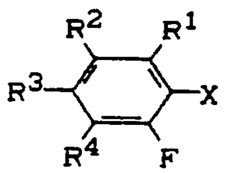

(IV)

in welcher
$R^1$, $R^2$, $R^3$, $R^4$ und X die oben angegebenen Bedeutungen haben,
mit 1,5-Dihydroxynaphthalin in Gegenwart eines Säureakzeptors, wie z. B. Natriumhydroxid oder Kaliumhydroxid, und in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen 20 °C und 150 °C umsetzt und nach üblichen Methoden aufarbeitet.

Die Halogen-benzol-Derivate sind durch die Formel (IV) allgemein definiert. In Formel (IV) haben $R^1$, $R^2$, $R^3$ und $R^4$ vorzugsweise bzw. insbesondere diejenigen Bedeutungen, die bereits im Zusammenhang mit der Beschreibung der erfindungsgemäßen Verbindungen der Formel (I) vorzugsweise bzw. als insbesondere bevorzugt für $R^1$, $R^2$, $R^3$ und $R^4$ angegeben wurden und X steht vorzugsweise für Chlor oder Fluor.

Als Beispiele für die Halogen-benzol-Derivate der Formel (IV) seien genannt:
3,4-Difluor-benzotrifluorid, 3,4,5-Trifluor-benzotrifluorid, 2,3,4,5-Tetrafluor-benzotrifluorid und 3-Cyano-4,5-difluor-benzotrifluorid.

Die Verbindungen der Formel (IV) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; US 4808750; FR-A 2 538 380 (Chem. Abstracts 102 (1985), 61914x)).

Die beim erfindungsgemäßen Verfahren (a) weiter als Ausgangsstoffe zu verwendenden Propionsäure-Derivate sind durch die Formel (III) allgemein definiert. In Formel (III) hat $R^5$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde und Y steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methyl-phenylsulfonyloxy.

Als Beispiele für die Verbindungen der Formel (III) seien genannt:
α-Chlor-, α-Brom- und α-Iod-propionsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy, α-Phenylsulfonyloxy- und α-(4-Methyl-phenylsulfonyloxy)-propionsäure-methylester, -ethylester, -propylester, -butylester, -isopropylester, -isobutylester und -sec-butylester.

Unter den genannten Verbindungen der Formel (III) sind jeweils die R-Isomeren, die S-Isomeren und die racemischen Gemische dieser Isomeren zu verstehen.

Die Ausgangsstoffe der Formel (III) sind bekannt und/oder können nach an sich bekannten Verfahren hergestellt werden (vgl. DE-OS 27 58 002, DE-OS 28 54 542).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und

Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Die beim erfindungsgemäßen Verfahren (b) als Ausgangsstoffe zu verwendenden Halogen-benzol-Derivate der Formel (IV) wurden bereits oben beschrieben.

Die beim erfindungsgemäßen Verfahren (b) weiter als Ausgangsstoffe zu verwendenden α-(5-Hydroxy-naphthalin-1-yl-oxy)-propionsäure-Derivate sind durch die Formel (V) allgemein definiert. In Formel (V) hat $R^5$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde.

Als Beispiele für die Ausgangsstoffe der Formel (V) seien genannt:
α-(5-Hydroxy-naphthalin-1-yl-oxy)-propionsäure-, -methylester, -ethylester, -propylester, -isopropylester, -butylester, -isobutylester und -sec-butylester.

Die Ausgangsstoffe der Formel (V) sind bekannt und/oder können nach an sich bekannten Verfahren hergestellt werden (vgl. JP 79/32477, zit. in Chem. Abstracts 91 (1979), 91510j).

Verfahren (b) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden. Aprotisch polare organische Lösungsmittel, wie z. B. Aceton, Acetonitril, Methylethylketon, Propionitril, Dimethylformamid, Dimethylacetamid, Dimethylsulfoxid, Sulfolan und N-Methylpyrrolidon werden besonders bevorzugt.

Verfahren (b) wird vorzugsweise in Gegenwart eines Säureakzeptors duchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man je Mol Halogen-benzol-Derivat der Formel (IV) im allgemeinen zwischen 0,5 und 2 Mol, vorzugsweise zwischen 0,7 und 1,5 Mol, α-(5-Hydroxy-naphthalin-1-yl-oxy)-propionsäure-Derivat der Formel (V) ein.

Umsetzung und Aufarbeitung können wie oben für Verfahren (a) angegeben durchgeführt werden.

Die beim erfindungsgemäßen Verfahren (c) als Ausgangsstoffe zu verwendenden Verbindungen sind durch die Formel (I) mit der Maßgabe, daß $R^5$ für Methoxy oder Ethoxy steht, allgemein definiert. In diesem Fall haben die Reste $R^1$, $R^2$, $R^3$ und $R^4$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (c) seien genannt:
α-(5-(2-Fluor-4-trifluormethyl-phenoxy)-, α-(5-(2,6-Difluor-4-trifluormethyl-phenoxy)- und α-(5-(2,3,6-Trifluor-4-trifluormethyl-phenoxy)-naphthalin-1-yl-oxy)-propionsäure-methylester und -ethylester.

Die oben beschriebenen Ausgangsstoffe der Formel (I) für Verfahren (c) sind erfindungsgemäße neue Verbindungen; sie können nach dem erfindungsgemäßen Verfahren (a) oder (b) hergestellt werden.

Verfahren (c) wird unter Verwendung von Alkalihydroxiden durchgeführt. Als Beispiel hierfür seien Lithiumhydroxid, Natriumhydroxid und Kaliumhydroxid genannt. Vorzugsweise wird Natriumhydroxid verwendet.

Verfahren (c) wird in Gegenwart von Wasser und gegebenenfalls in Gegenwart eines organischen Lösungsmittels durchgeführt. Als organische Lösungsmittel werden vorzugsweise Alkohole, wie z. B. Methanol oder Ethanol eingesetzt.

Zum Ansäuern werden bei Verfahren (c) die üblichen Mineralsäuren, wie z. B. Salzsäure oder Schwefelsäure, verwendet.

Die Reaktionstemperaturen können bei dem erfindungsgemäßen Verfahren (c) in einem größeren Bereich variiert werden. Im allgemeinen arbeitet man bei Temperaturen zwischen 10 °C und 150 °C, vorzugsweise bei Temperaturen zwischen 20 °C und 100 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung von Verfahren (c) werden je Mol Ausgangsverbindung der Formel (I) im allgemeinen zwischen 0,1 und 10 Mol, vorzugsweise zwischen 0,5 und 2 Mol, Alkalihydroxid eingesetzt. Die Reaktionskomponenten werden im allgemeinen bei Raumtemperatur zusammengegeben und das Reaktionsgemisch wird gegegenenfalls bei erhöhter Temperatur, bis zum Reaktionsende gerührt. Das - gegebenenfalls nach Einengen, Abkühlen und Ansäuern - kristallin anfallende Reaktionsprodukt kann durch Absaugen isoliert werden.

Die beim erfindungsgemäßen Verfahren (d) als Ausgangsstoffe zu verwendenden Verbindungen sind durch die Formel (I) mit der Maßgabe, daß $R^5$ für Hydroxy steht, allgemein definiert. In diesem Fall haben die Reste $R^1$, $R^2$, $R^3$ und $R^4$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (d) seien genannt:

α-(5-(2-Fluor-4-trifluormethyl-phenoxy)-, α-(5-(2,6-Difluor-4-trifluormethyl-phenoxy)- und α-(5-(2,3,6-Trifluor-4-trifluormethyl-phenoxy)-naphthalin-1-yl-oxy)-propionsäure.

Die oben beschriebenen Ausgangsstoffe der Formel (I) für Verfahren (d) sind erfindungsgemäße, neue Verbindungen; sie können nach dem erfindungsgemäßen Verfahren (c) hergestellt werden.

Verfahren (d) wird unter Verwendung eines Chlorierungsmittels durchgeführt. Es können die üblichen Mittel für die Umsetzung von Carbonsäuren zur Carbonsäurechloriden eingesetzt werden. Als Beispiele hierfür seien Phosgen, Thionylchlorid, Phosphorylchlorid und Benzotrichlorid genannt. Vorzugsweise wird Thionylchlorid als Chlorierungsmittel verwendet.

Verfahren (d) wird gegebenenfalls in Gegenwart eines Katalysators duchgeführt. Es können die für die Herstellung von Säurechloriden aus Säuren üblichen Katalysatoren, wie z. B. Pyridin oder Dimethylformamid verwendet werden.

Verfahren (d) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsgemäßen Verfahren (d) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (d) wird im allgemeinen bei Normaldruck durchgeführt.

Zur Durchführung von Verfahren (d) werden je Mol Ausgangsverbindung der Formel (I) im allgemeinen zwischen 1 und 100 Mol, vorzugsweise zwischen 2 und 50 Mol, Chlorierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden, aber auch ohne weitere Reinigung für Folgeumsetzungen eingesetzt werden.

Die beim erfindungsgemäßen Verfahren (e) als Ausgangsstoffe zu verwendenden Verbindungen sind durch die Formel (I) mit der Maßgabe, daß $R^5$ für Chlor steht allgemein definiert. In diesem Fall haben die Reste $R^1$, $R^2$, $R^3$ und $R^4$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe zu Verfahren (e) seien genannt:
α-(5-(2-Fluor-4-trifluormethyl-phenoxy)-, α-(5-(2,6-Difluor-4-trifluormethyl-phenoxy)- und α-(5-(2,3,6-Trifluor-4-trifluormethyl-phenoxy)-naphthalin-1-yl-oxy)-propionsäure-chlorid.

Die oben beschriebenen Ausgangsstoffe der Formel (I) für Verfahren (e) sind erfindungsgemäße, neue Verbindungen; sie können nach dem erfindungsgemäßen Verfahren (d) hergestellt werden.

Die beim erfindungsgemäßen Verfahren (e) weiter als Ausgangsstoffe einzusetzenden Verbindungen sind durch die Formel (VI) allgemein definiert. In Formel (VI) hat $R^5$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt angegeben wurde.

Als Beispiele für die Ausgangsstoffe der Formel (VI) seien genannt:
Methylamin, Ethylamin, Propylamin, Isopropylamin, Anilin, Cyanamid, Dimethylamin, Diethylamin, Hydroxylamin, O-Methylhydroxylamin, Hydrazin, Methylsulfonylhydrazin, Methanol, Ethanol, Propanol, Isopropanol, Butanol, Isobutanol, sec-Butanol, 2-Methoxy-ethanol, 2-Ethoxy-ethanol, 2-Methylthio-ethanol, 2-Ethylthio-ethanol, 2-Benzyloxy-ethanol, 3-Benzyloxy-propanol, 2-Benzylthio-ethanol, Hydroxymethanphosphonsäure-diethylester und -dimethylester, 1-Hydroxy-ethan-phosphonsäure-dimethylester und -diethylester, 1-Hydroxy-1-phenyl-methanphosphonsäure-dimethylester und -diethylester, Acetonoxim, 3-Hydroxyfuran, Furfurylalkohol, Perhydrofurfurylalkohol, Milchsäure-methylester und -ethylester und Glycolsäuremethylester und -ethylester.

Diese Verbindungen sind bekannte Synthesechemikalien.

Verfahren (e) wird vorzugsweise unter Verwendung eines Verdünnungsmittels durchgeführt. Es kommen vor allem diejenigen Verdünnungsmittel in Betracht, die bereits bei der Beschreibung des erfindungsgemäßen Verfahrens (a) genannt wurden.

Verfahren (e) wird vorzugsweise in Gegenwart eines Säureakzeptors durchgeführt. Es kommen vor allem diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (e) in einem größeren Bereich variiert werden. Im allgemeinen arbeitet man bei Temperaturen zwischen -20 °C und +100 °C, vorzugsweise bei Temperaturen zwischen 0 °C und 50 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Umsetzungen werden im allgemeinen in einem geeigneten Verdünnungsmittel in Gegenwart eines Säureakzeptors durchgeführt und das Reaktionsgemisch wird mehrere Stunden bei der jeweils erforderlichen Temperatur gerührt.

Die Aufarbeitung erfolgt bei dem erfindungsgemäßen Verfahren (e) jeweils nach üblichen Methoden (vgl. die Herstellungsbeispiele).

Die beim erfindungsgemäßen Verfahren (f) als Ausgangsstoffe zu verwendenden Verbindungen sind durch die Formel (I) mit der Maßgabe, daß $R^5$ für Hydroxy steht, allgemein definiert. In diesem Fall haben die Reste $R^1$, $R^2$, $R^3$ und $R^4$ vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Als Beispiele für die Ausgangsstoffe der Formel (I) zu Verfahren (f) seien genannt:
α-(5-(2-Fluor-4-trifluormethyl-phenoxy)-, α-(5-(2,6-Difluor-4-trifluormethyl-phenoxy)- und α-(5-(2,3,6-Trifluor-4-trifluormethyl-phenoxy)-naphthalin-1-yl-oxy)-propionsäure.

Die oben beschriebenen Ausgangsstoffe der Formel (I) für Verfahren (f) sind erfindungsgemäße, neue Verbindungen; sie können nach dem erfindungsgemäßen Verfahren (c) hergestellt werden.

Die beim erfindungsgemäßen Verfahren (f) weiter als Ausgangsstoffe einzusetzenden Verbindungen sind durch die Formel (VII) allgemein definiert. In Formel (VII) steht vorzugsweise $R^6$ für $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl oder $C_1$-$C_4$-Alkoxy-carbonyl-$C_1$-$C_2$-alkyl.

Die Ausgangstoffe der Formel (VII) sind bekannte Synthesechemikalien.

Verfahren (f) wird vorzugsweise unter Verwendung eines Vedünnungsmittels durchgeführt. Als solche kommen insbesondere die Hydroxyverbindungen der Formel (VII) in Betracht, welche bei Verfahren (f) als Reaktionskomponenten eingesetzt werden.

Verfahren (f) wird vorzugsweise in Gegenwart eines Katalysators durchgeführt. Als solche kommen vorzugsweise starke Säuren, wie z.B. Schwefelsäure, Salzsäure, p-Toluolsulfonsäure und Methansulfonsäure in Betracht.

Die Reaktionstemperaturen können bei dem erfindungsgemäßen Verfahren (f) in einem größeren Bereich variiert werden. Im allgemeinen arbeitet man bei Temperaturen zwischen 0°C und 150°C, vorzugsweise bei Temperaturen zwischen 20°C und 100°C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung von Verfahren (f) setzt man die Ausgangsverbindung der Formel (VII) im allgemeinen im Überschuß ein, so daß sie auch als Verdünnungsmittel dient.

Umsetzung und Aufarbeitung können nach üblichen Methoden durchgeführt werden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung vor allem von monokotylen Unkräutern in monokotylen und dikotylen Kulturen sowohl im Vorauflauf- als auch im Nachauflauf-Verfahren.

In gewissem Umfang zeigen die Verbindungen der Formel (I) auch insektizide Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und

fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); 4-Amino-benzolsulfonyl-methylcarbonat (ASULAM); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amin-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexylthiolcarbamat (CYCLOATE); 2-[4-(2,4-Dichlorphenoxy)-phenoxy]-propionsäure, deren Methyl-oder deren Ethylester (DICLOFOP); 2-[(2-Chlorphenyl)-methyl]-4,4-dimethylisoxazolidin-3-on (DIMETHAZONE); N,N-Di-n-propylthiocarbamidsäure-S-ethylester (EPTAME); 4-Amino-6-t-butyl-3-ethylthio-1,2,4-triazin-5(4H)-on (ETHIOZIN); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethylphenoxy)-N-methylsulfonyl-2-nitrobenzamid (FOMESAFEN); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenylcarbamat (PROPHAM); 0-(6-Chlor-3-phenyl-pyridazin-4-yl)-S-octyl-thiocarbonat (PYRIDATE); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

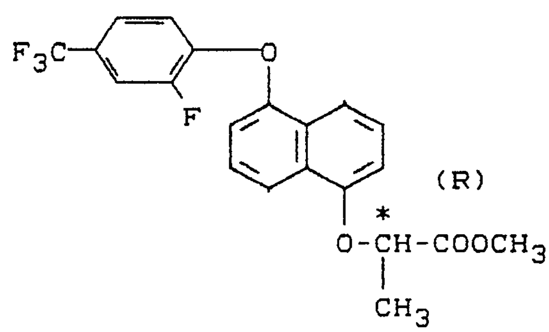

(Verfahren (a))

Eine Mischung aus 1,3 g (4 mMol) 5-(2-Fluor-4-trifluormethyl-phenoxy)-1-naphthol, 0,5 g (4 mMol) (S)-α-Chlorpropionsäure-methylester, 0,6 g (4,4 mMol) Kaliumcarbonat und 50 ml Acetonitril wird 3 Tage bei 20 °C und 6 Stunden unter Rückfluß gerührt, dann eingeengt, mit Wasser/Methyl-tert-butylether geschüttelt, die organische Phase abgetrennt, mit Natriumsulfat getrocknet und filtriert. Vom Filtrat wird das Lösungsmittel im Wasserstrahlvakuum sorgfältig abdestilliert.

Man erhält 1,2 g (73,5% der Theorie) (R)-α-(5-(2-Fluor-4-trifluormethyl-phenoxy)-naphthalin-1-yl-oxy)-propionsäure-methylester als amorphen Rückstand.

$^1$H-NMR ($CDCl_3$, δ): 4,98 ppm (q)

Beispiel 2

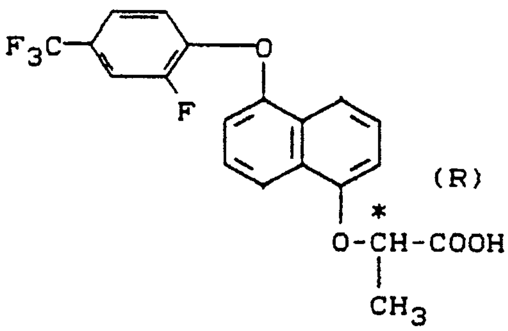

(Verfahren (c))

Eine Mischung aus 15,8 g (39 mMol) (R)-α-(5-(2-Fluor-4-trifluormethyl-phenoxy)-naphthalin-1-yl-oxy)-propionsäure-methylester, 2,0 g (49 mMol) Natriumhydroxid und 150 ml Wasser wird 15 Stunden unter Rückfluß erhitzt, nach Abkühlen mit 2N-Salzsäure angesäuert und 30 Minuten bei ca. 10 °C gerührt. Das kristallin angefallene Produkt wird durch Absaugen isoliert.

Man erhält 14,6 g (95% der Theorie) (R)-α-(5-(2-Fluor-4-trifluormethyl-phenoxy)-naphthalin-1-yl-oxy)-propionsäure vom Schmelzpunkt 166 °C.

Beispiel 3

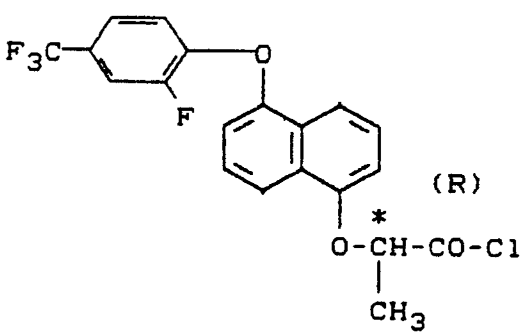

(Verfahren (d))

Eine Mischung aus 1,3 g (3,3 mMol) (R)-α-(5-(2-Fluor-4-trifluormethyl-phenoxy)-naphthalin-1-yl-oxy)-propionsäure und 20 ml Thionylchlorid wird 15 Stunden unter Rückfluß erhitzt. Anschließend wird das überschüssige Thionylchlorid im Dampfstrahlvakuum (ca. 5 mbar) sorgfältig abdestilliert.

Man erhält (R)-α-(5-(2-Fluor-4-trifluormethyl-phenoxy)-naphthalin-1-yl-oxy)-propionsäurechlorid als amorphen Rückstand. Das Produkt wird ohne weitere Reinigung für weitere Umsetzungen (vgl. Beispiel 4) eingesetzt.

Beispiel 4

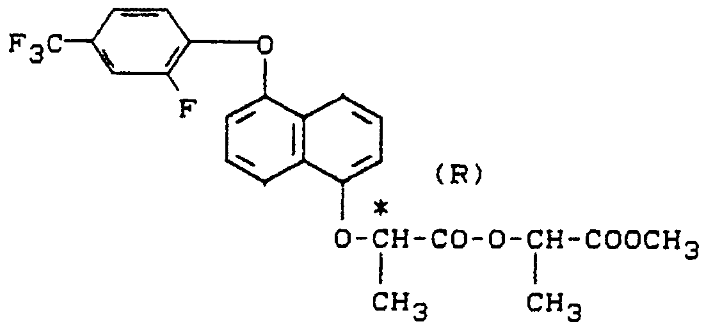

(Verfahren (e))

Eine Mischung aus 1,4 g (3,3 mMol) (R)-α-(5-(2-Fluor-4-trifluormethyl-phenoxy)-naphthalin-1-yl-oxy)-propionsäurechlorid, 0,4 g (3,5 mMol) Milchsäuremethylester, 0,5 g (5 mMol) Triethylamin und 50 ml Methylenchlorid wird 15 Stunden bei 20° C gerührt und anschließend eingeengt.

Der Rückstand wird mit Wasser/Methyl-tert-butylether geschüttelt, die organische Phase abgetrennt, nacheinander mit 2N-Salzsäure und mit 5%iger Natriumhydrogencarbonat-Lösung gewaschen mit Natriumsulfat getrocknet und filtrier. Vom Filtrat wird das Lösungsmittel abdestilliert und der Rückstand durch Säulenchromatographie (Toluol/Kieselgel) gereinigt.

Man erhält 1,1 g (69% der Theorie) (R)-α-(5-(2-Fluor-4-trifluormethyl-phenoxy)-naphthalin-1-yl-oxy)-propionsäure-(1-methoxycarbonyl-ethylester) als amorphe Masse.

$^1$H-NMR ($CDCl_3$, δ): 5,5 ppm (q)

Beispiel 5

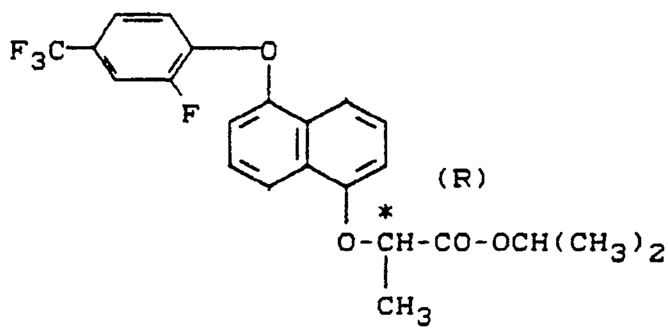

(Verfahren (f))

Eine Mischung aus 3,9 g (10 mMol) (R)-α-(5-(2-Fluor-4-trifluormethyl-phenoxy)-naphthalin-1-yl-oxy)-propionsäure, 0,2 ml konzentrierter Schwefelsäure und 200 ml Isopropanol wird 15 Stunden unter Rückfluß erhitzt, anschließend eingeengt, mit 5%iger Natriumhydrogencarbonat-Lösung verrührt und mit Methyl-tert-butylether geschüttelt. Die organische Phase wird abgetrennt, mit Natriumsulfat getrocknet und filtriert. Vom Filtrat wird das Lösungsmittel im Wasserstrahlvakuum sorgfältig abdestilliert.

Man erhält 3,5 g (80% der Theorie) (R)-α-(5-(2-Fluor-4-trifluormethyl-phenoxy)-naphthalin-1-yl-oxy)-propionsäure-isopropylester als amorphen Rückstand.

$^1$H-NMR ($CDCl_3$, δ): 4,92 ppm (q)

Analog zu den Beispielen 1 bis 5 sowie entsprechend der allgemeinen Beschreibung der erfindungsgemäßen Herstellungsverfahren können beispielsweise auch die in der nachstehenden Tabelle 1 aufgeführten

Verbindungen der Formel (I) hergestellt werden.

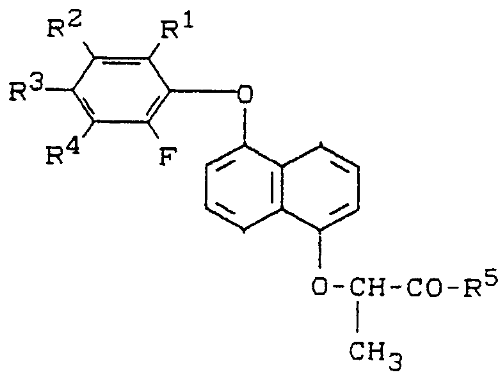

(I)

Tabelle 1: Beispiele für die Verbindungen der Formel (I)

| Bsp.-Nr. | $R^1$ | $R^2$ | $R^3$ | $R^4$ | $R^5$ | Physikalische Daten |
|---|---|---|---|---|---|---|
| 6 | H | H | $CF_3$ | H | $OC_4H_9$-n | $\delta$ = 4,97 ppm |
| 7 | H | H | $CF_3$ | H | $OC_2H_5$ | $\delta$ = 4,95 ppm |
| 8 | H | H | $CF_3$ | H | 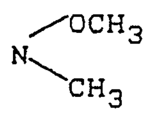 | Fp.: 117° C |
| 9 | H | H | $CF_3$ | H | $-O-CH_2$ 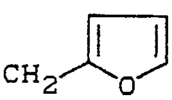 | $\delta$ = 5,17 ppm |

Tabelle 1: Fortsetzung

| Bsp.-Nr. | $R^1$ | $R^2$ | $R^3$ | $R^4$ | $R^5$ | Physikalische Daten |
|---|---|---|---|---|---|---|
| 10 | H | H | $CF_3$ | H | 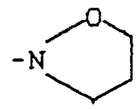 | (amorph) |
| 11 | H | H | $CF_3$ | H | $-O-(CH_2)_3-O-CH_2-$ 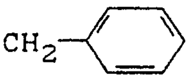 | (amorph) |
| 12 | H | H | $CF_3$ | H | $-O-(CH_2)_2-O-N=C$ $CH_3$ / $CH_3$ | (amorph) |
| 13 | H | H | $CF_3$ | H | $-O-CH_2-$ 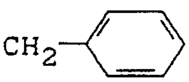 | δ = 5,00 ppm |
| 14 | H | H | $CF_3$ | H | $-O-CH_2-C\equiv CH$ | δ = 4,78 ppm |
| 15 | H | H | $CF_3$ | H | $-S-CH_3$ | δ = 5,08 ppm |

Ausgangsstoffe der Formel (II):

Beispiel (II-1)

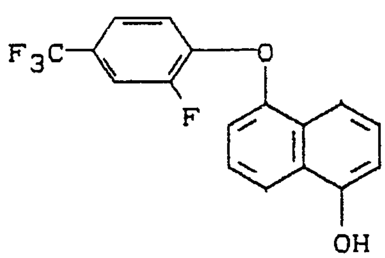

3,4 g (0,06 Mol) Kaliumhydroxid (Pulver) werden zu einer Lösung von 16,0 g (0,10 Mol) 1,5-Dihydroxynaphthalin in 150 ml Dimethylsulfoxid gegeben und das Gemisch wird 60 Minuten bei 20°C gerührt. Dann werden 6,6 g (0,036 Mol) 3,4-Difluor-benzotrifluorid dazugegeben; das Reaktionsgemisch wird 20 Stunden bei 60°C und dann 50 Stunden bei 80°C gerührt und anschließend im Dampfstrahlvakuum eingeengt. Der Rückstand wird mit 2N-Salzsäure verrührt und mit Essigsäureethylester geschüttelt. Die organische Phase wird abgetrennt, mit Natriumsulfat getrocknet und filtriert. Vom Filtrat wird das Lösungsmittel im Wasserstrahlvakuum abdestilliert, der Rückstand in Toluol aufgekocht, über Kieselgel filtriert, eingeengt, mit Ligroin verrieben und abgesaugt.

Man erhält 2,4 g (21% der Theorie) 5-(2-Fluor-4-trifluormethyl-phenoxy)-1-naphthol als amorphes Produkt.

Anwendungsbeispiele:

In den folgenden Anwendungsbeispielen wird die nachstehend aufgeführte Verbindung als Vergleichssubstanz herangezogen:

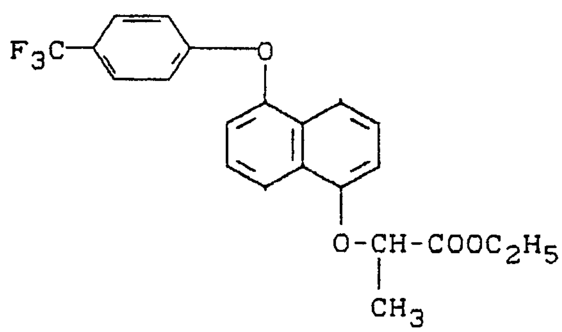

(A)

<ℓΨ>α-(5-(4-Trifluormethyl-phenoxy)-naphthalin-1-yl-oxy)-propionsäure-ethylester (bekannt aus EP-A 309864/Beispiel 2).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine bis zu 90 % höhere Wirksamkeit und eine Überlegenheit in der Nutzpflanzenselektivität gegenüber dem Stand der Technik zeigt in diesem Test z.B. die Verbindung gemäß Herstellungsbeispiel 1, 7, 10, 11 und 12.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine bis zu 80 % höhere Wirksamkeit gegenüber dem Stand der Technik zeigt in diesem Test z.B. die Verbindung gemäß Herstellungsbeispiel 1.

Beispiel C

Lösungsmittel: 5 Gewichtsteile Aceton
Emulgator : 1 Gewichtsteil Benzyloxypolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat auf die gewünschte Konzentration.

Pflanzgefäße (Oberfläche 1/5000 Ar) werden mit Boden aus einem Reisfeld gefüllt. Zwei Reispflanzen (Sorte: Kinmaze) werden im 2-3 Blattstadium (ca. 10 cm hoch) in die Gefäße verpflanzt. Samen von Echinochloa crus galli und/oder Monochoria vaginalis und/oder kleine Rhizomabschnitte von Eleocharis acicularis L. werden in die feucht gehaltene Erde ausgesät. 2 Tage nach dem Verpflanzen des Reises wird der Boden bis zu einer Wassertiefe von 3 cm überstaut. Die Wirkstoffzubereitung wird auf die Wasseroberfläche ausgebracht. Die Wirkstoffkonzentration in der Zubereitung spielt keine Rolle, entscheidend ist nur die Aufwandmenge des Wirkstoffs pro Flächeneinheit.

Nach der Anwendung des Wirkstoffs wird für 2 Tage durch die Pflanzengefäße ein vertikal absteigender Wasserstrom mit einer Geschwindigkeit von 2-3 cm pro Tag eingestellt. Danach werden die Testansätze unter Überflutungsbedingungen gehalten, wobei die Wassertiefe 3 cm beträgt. Nach 4 Wochen wird der Schädigungsgrad der Pflanzen bonitiert in % Schädigung (bzw. Unkrautwirkung) im Vergleich zu einer unbehandelten Kontrolle.

Es bedeuten:

0 % = keine Wirkung (wie unbehandelte Kontrolle)
100 % = totale Vernichtung.

In diesem Test zeigen z.B. die Verbindungen gemäß Herstellungsbeispiel 7, 10, 11 und 12 eine bis zu 100%ige Aktivität gegenüber den Unkräutern.

**Patentansprüche**

1. Fluor-substituierte $\alpha$-(5-Aryloxy-naphthalin-1-yl-oxy)-propionsäure-Derivate der allgemeinen Formel (I)

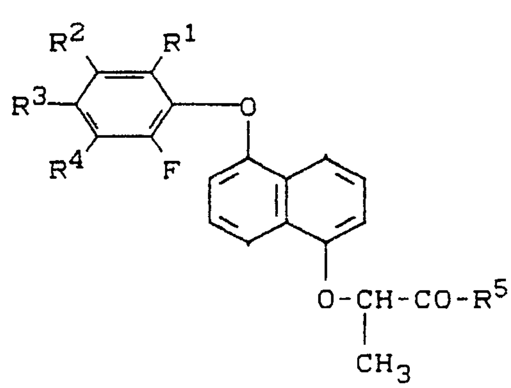

(I)

in welcher

$R^1$ für Wasserstoff, Fluor oder Cyano steht,

$R^2$ für Wasserstoff, Fluor oder Chlor steht,

$R^3$ für Fluor, Chlor, Cyano, Trifluormethyl oder Trifluormethylsulfonyl steht,

$R^4$ für Wasserstoff, Fluor oder Chlor steht und

$R^5$ für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, N-($C_1$-$C_6$-Alkoxy)-N-($C_1$-$C_4$-alkyl)-amino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^6$ steht, worin

$R^6$ für einen gegebenenfalls durch Fluor und/oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino-oxy-C1-C4-alkyl, $C_2$-$C_4$-Alkylidenamino oder für ein Ammonium-, ein $C_1$-$C_4$-Alkylammonium-, ein Natrium-, Kalium- oder Calcium-äquivalent steht, oder für die Gruppierung

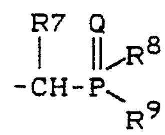

steht, worin

$R^7$ für Wasserstoff, $C_1$-$C_4$-Alkyl, Phenyl, Furyl, Thienyl oder Pyridyl steht,

$R^8$ für $C_1$-$C_4$-Alkyl oder $C_1$-$C_4$-Alkoxy steht,

$R^9$ für $C_1$-$C_4$-Alkoxy steht und

Q für Sauerstoff oder Schwefel steht,

oder

$R^6$ für die Gruppierung $-(CH_2)_n-R^{10}$ steht, worin

n für die Zahlen 0, 1 oder 2 steht und

$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Isoxazolidinyl, Pyridinyl oder Pyrimidinyl steht,

gefunden.

2. Verbindungen der Formel (I), gemäß Anspruch 1 in welcher

$R^1$ für Wasserstoff oder Fluor steht,

$R^2$ für Wasserstoff oder Fluor steht,

$R^3$ für Trifluormethyl oder Trifluormethylsulfonyl steht,

$R^4$ für Wasserstoff oder Fluor steht und

$R^5$ für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkylsulfonylamino, Phenylsulfonylamino, Hydroxyamino, Cyanamino, $C_1$-$C_4$-Alkoxyamino, N-($C_1$-$C_4$-Alkoxy)-N-($C_1$-$C_3$-alkyl)-amino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung -O-$R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl, $C_2$-$C_4$-Alkylaminooxy-$C_2$-$C_3$-alkyl oder für ein Ammonium-, $C_1$-$C_3$-Alkylammonium-, Natrium-, oder Kalium-äquivalent steht, oder für die Gruppierung

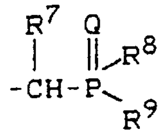

steht,
worin

$R^7$ für Wasserstoff, Methyl, Phenyl, Furyl, Thienyl oder Pyridyl steht,
$R^8$ für Methoxy oder Ethoxy steht,
$R^9$ für Methoxy oder Ethoxy steht und
Q für Sauerstoff oder Schwefel steht

oder

$R^6$ für die Gruppierung $(-CH_2-)_n-R^{10}$ steht, worin
n für die Zahlen 0, 1 oder 2 steht und
$R^{10}$ für einen gegebenenfalls durch Chlor und/oder Methyl subtituierten heterocyclischen Rest aus der Reihe Furyl, Tetrahydrofuryl, Thienyl, Perhydropyranyl, Oxazolyl, Thiazolyl, Isoxazolidinyl und Dioxolanyl steht.

**3.** Verbindungen der Formel (I), gemäß Anspruch 1, in welcher

$R^1$, $R^2$ und $R^4$ für Wasserstoff stehen,
$R^3$ für Trifluormethyl steht und
$R^5$ für Chlor, Hydroxy, Methoxy, Ethoxy, Propoxy, Isopropoxy, n-Butoxy, Isobutoxy, sec.-Butoxy, t-Butoxy, Methylethoxy, Ethoxyethoxy, Benzyloxyethoxy, Benzyloxypropoxy, Methoxycarbonylethoxy, iso-Propylidenamino-oxy-ethoxy oder Ethoxycarbonylethoxy steht.

**4.** Verfahren zur Hertellung von fluor-substituierten $\alpha$-(5-Aryloxy-naphthalin-1-yl-oxy)-propionsäure-Derivatender Formel (I), dadurch gekennzeichnet, daß man

(a) fluor-substituierte 5-Aryloxy-1-naphthole der all-gemeinen Formel (II)

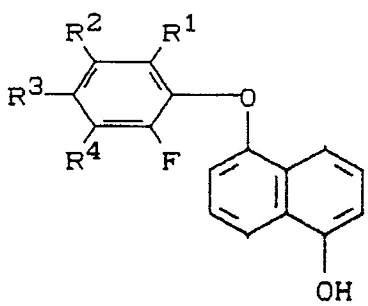

(II)

in welcher

$R^1$, $R^2$, $R^3$ und $R^4$ die in Anspruch 1 angegebenen Bedeutungen haben,

mit Propionsäure-Derivaten der allgemeinen Formel (III)

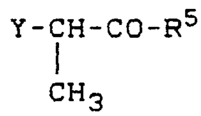

(III)

in welcher

$R^5$ die in Anspruch 1 angegebene Bedeutung hat und
Y für eine nucleophile Abgangsgruppe steht,

gegebenenfalls in Gegenwart eines Säureakzeptors und gegebenenfalls in Gegenwart eines Verdünnungsmittels umsetzt, oder

(b) Halogen-benzol-Derivate der allgemeinen Formel (IV)

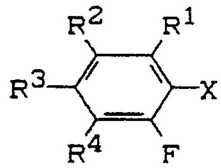

(IV)

in welcher

$R^1$, $R^2$, $R^3$ und $R^4$ die oben angegebenen Bedeutungen haben und

X für Halogen steht,

mit α-(5-Hydroxy-naphthalin-1-yl-oxy)-propionsäure-Derivaten der allgemeinen Formel (V)

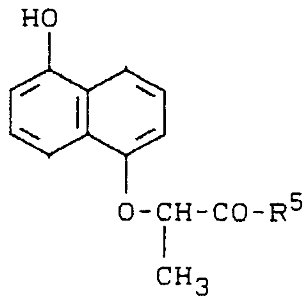

(V)

in welcher

$R^5$ die oben angegebene Bedeutung hat,

gegebenenfalls in Gegenwart eines Säureakzeptors und gegebenenfalls in Gegenwart eines Verdünnungsmittels umsetzt, oder

(c) für den Fall, daß $R^5$ für Hydroxy steht und die Reste $R^1$ bis $R^4$ die oben angegebenen Bedeutungen haben, Verbindungen der allgemeinen Formel (I), in welcher $R^5$ für Methoxy oder Ethoxy steht und die Reste $R^1$ bis $R^4$ die oben angegebenen Bedeutungen haben, mit einem Alkalihydroxid in Gegenwart von Wasser und gegebenenfalls in Gegenwart eines organischen Lösungsmittels umsetzt und dann - gegebenenfalls nach Einengen - mit einer Mineralsäure ansäuert, oder

(d) für den Fall, daß $R^5$ für Chlor steht und die Reste $R^1$ bis $R^4$ die oben angegebenen Bedeutungen haben, Verbindungen der allgemeinen Formel (I), in welcher $R^5$ für Hydroxy steht und die Reste $R^1$ bis $R^4$ die oben angegebenen Bedeutungen haben,

mit einem Chlorierungsmittel gegebenenfalls in Gegenwart eines Katalysators und gegebenenfalls in Gegenwart eines Verdünnungsmittels umsetzt, oder

(e) für den Fall, daß $R^5$ mit Ausnahme von Chlor die oben angegebene Bedeutung hat und die Reste $R^1$ bis $R^4$ die oben angegebenen Bedeutungen haben, Verbindungen der allgemeinen Formel (I), in welcher $R^5$ für Chlor steht und die Reste $R^1$ bis $R^4$ die oben angegebenen Bedeutungen haben,

mit Verbindungen der allgemeinen Formel (VI)

$H-R^5$ (VI)

in welcher

$R^5$ mit Ausnahme von Chlor die oben angegebene Bedeutung hat,

gegebenenfalls in Gegenwart eines Säureakzeptors und gegebenenfalls in Gegenwart eines Verdünnungsmittels umsetzt, oder

(f) für den Fall, daß $R^5$ für die Gruppierung $-O-R^6$ steht, worin $R^6$ mit Ausnahme von Ammonium, Alkylammonium, Alkalimetall und Erdalkalimetall die oben angegebene Bedeutung hat und die Reste $R^1$ bis $R^4$ die oben angegebenen Bedeutungen haben, Verbindungen der allgemeinen Formel (I), in welcher $R^5$ für Hydroxy steht und die Reste $R^1$ bis $R^4$ die oben angegebenen Bedeutungen haben,

mit Hydroxyverbindungen der allgemeinen Formel (VII)

$HO-R^6$ (VII)

in welcher

$R^6$ mit Ausnahme von Ammonium, Alkylammonium, Alkalimetall und Erdalkalimetall die oben angegebenen Bedeutungen hat,

gegebenenfalls in Gegenwart eines Katalysators und gegebenenfalls in Gegenwart eines Verdünnungsmittels umsetzt.

**5.** Herbizide Mittel, gekennzeichnet durch einen Gehalt an mindestens einem Fluor-substiutuiertem α-(5-Arylox-naphthalin-1-yl-oxy)propionsäure-Derivat der Formel (I) gemäß den Ansprüchen 1 bis 4.

**6.** Verfahren zur Bekämpfung von Unkräutern, dadurch gekennzeichnet, daß man Fluor-substituierten α-(5-Aryloxy-naphthalin-1-yl-oxy)propionsäure-Derivate der Formel (I) gemäß den Ansprüchen 1 bis 4 auf die Unkräutern und/oder ihren Lebensraum einwirken läßt.

**7.** Verwendung von Fluor-substituierten α-(5-Aryloxynaphthalin-1-yl-oxy)propionsäure-Derivaten der Formel (I) gemäß den Ansprüchen 1 bis 4 zur Bekämpfung von Unkräutern.

**8.** Verfahren zur Herstellung von herbiziden Mitteln, dadurch gekennzeichnet, daß man Fluor-substituierten α-(5-Aryloxy-naphthalin-1-yl-oxy)-propionsäure-Derivate der Formel (I) gemäß Ansprüchen 1 bis 4 mit Streckmitteln und/oder oberflächenaktiven Substanzen vermischt.

**9.** Fluor-substituierte 5-Aryloxy-1-naphthole der allgemeinen Formel (II)

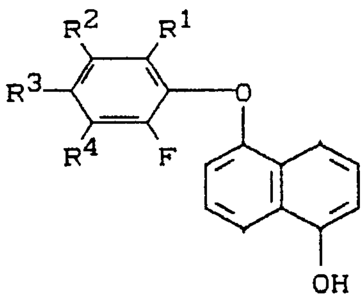

(II)

in welcher

$R^1$, $R^2$, $R^3$ und $R^4$ die in Anspruch 1 angegebene Bedeutung haben.

**10.** Verfahren zur Herstellung von Verbindungen der Formel (II), gemäß Anspruch 9, dadurch gekennzeichnet, daß man Fluor-benzol-Derivate der allgemeinen Formel (IV)

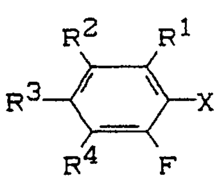

(IV)

in welcher

$R^1$, $R^2$, $R^3$ und $R^4$ die in Anspruch 1 angegebenen Bedeutungen haben, und

X für Halogen steht,

mit 1,5-Dihydroxynaphthalin in Gegenwart eines Säureakzeptors und in Gegenwart eines Verdünnungsmittels bei Temperaturen zwischen 20 °C und 150 °C umsetzt und nach üblichen Methoden aufarbeitet.